# EUROPEAN PATENT APPLICATION

(11) **EP 2 264 070 A1**
(43) Date of publication of application: **22.12.2010**
(21) Application number: 10175810.0
(22) Date of filing: 02.08.2000
(51) Int. Cl.: C07K 16/28, A61K 39/395, A61P 35/00

(54) **Treatment of intermediate-and high-grade non-hodgkins lymphoma with anti-CD20 antibody**

(30) Priority: 11.08.1999 US 148286 P; 28.07.2000 US 628187
(62) Divisional of application: 00955249.8
(71) Applicant: Biogen-Idec Inc., Cambridge, MA 02142 (US)
(72) Inventor: White, Christine A., Rancho Santa Fe, CA 92067 (US); Grillo-Lopez, Antonio, Rancho Santa Fe, CA 92067 (US)
(74) Representative: Walton, Seán Malcolm

(57) **Abstract**

This invention discloses methods for the treatment of intermediate- and high-grade non-Hodgkins lymphomas comprising administration of anti-CD20 antibodies.

## Description

### Field of the Invention

The present invention concerns methods of treating intermediate- and high-grade non-Hodgkins lymphomas, and lymphomas associated with a high level of bone marrow involvement with anti-CD20 monoclonal antibodies and fragments thereof.

### Background of the Invention

Non-Hodgkin's lymphoma is characterized by the malignant growth of B lymphocytes. According to the American Cancer Society, an estimated 54,000 new cases will be diagnosed, 65% of which will be classified as intermediate- or high-grade lymphoma. Patients diagnosed with intermediate-grade lymphoma have an average survival rate of 2-5 years, and patients diagnosed with high-grade lymphoma survive an average of 6 months to 2 years after diagnosis.

Intermediate- and high-grade lymphomas are much more aggressive at the time of diagnosis than are low-grade lymphomas, where patients may survive an average of 5-7 years with conventional therapies. Intermediate- and high-grade lymphomas are often characterized by large extranodal bulky tumors and a large number of circulating cancer cells, which often infiltrate the bone marrow of the patient.

Conventional therapies have included chemotherapy and radiation, possibly accompanied by either autologous or allogeneic bone marrow or stem cell transplantation if a suitable donor is available, and if the bone marrow contains too many tumor cells upon harvesting. While patients often respond to conventional therapies, they usually relapse within several months.

A relatively new approach to treating non-Hodgkin's lymphoma has been to treat patients with a monoclonal antibody directed to a protein on the surface of cancerous B cells. The antibody may be conjugated to a toxin or radiolabel thereby affecting cell death after binding. Alternatively, an antibody may be engineered with human constant regions such that human antibody effector mechanisms are generated upon antibody binding which result in apoptosis or death of the cell.

One antibody currently being investigated for the treatment of intermediate- and high-grade lymphomas is Oncolym® (¹³¹I-Lym-1)(Techniclone Corp.), which is a murine IgG2a monoclonal antibody which recognizes the HLA-Dr10 protein which is present on the surface of over 80% of lymphoma cells. Only 2% of normal B cells (noncancerous) express the HLA-Dr10 molecule. Oncolym® is conjugated to a Iodine-[131] (¹³¹I), a radioactive isotope of iodine which emits beta irradiation for a distance of several millimeters, and is thereby thought to be an effective approach to targeting the outer rim of tumors and halting the progression of bulky disease.

However, a potential disadvantage in using Oncolym® in advanced forms of non-Hodgkin's lymphoma is that such lymphomas are often characterized by bone marrow involvement. Thus, administration of a radiolabeled antibody to such patients often results in unwanted myelosuppression and damage to healthy progenitor cells.

It would be advantageous if alternative therapies and other monoclonal antibodies could be administered to patients with intermediate- and high-grade lymphomas which circumvent some of the deficiencies associated with current treatments and decrease the frequency of relapse.

### Summary of Invention

The present invention concerns the use of anti-CD20 antibodies for the treatment of intermediate- and high-grade lymphomas, particularly those which are characterized by bone marrow involvement and bulky disease. In particular, the present inventors have surprisingly found that Rituximab®, a chimeric anti-CD20 antibody already approved for the treatment of low-grade follicular non-Hodgkin's lymphoma, may be effective to treat more aggressive lymphomas as well.

### Detailed Description of the Invention

The present invention relates to methods for treating or alleviating the symptoms of intermediate- or high-grade non-Hodgkins lymphoma, or other lymphomas associated with a high degree of bone marrow involvement, comprising administering to a patient a therapeutically effective amount of an anti-CD20 antibody or other lymphoma cell depleting antibody, e.g. anti-CD19 and anti-CD22 antibodies, or a therapeutically effective fragment thereof. The present invention also includes administering anti-CD20 antibodies, or other lymphoma cell depleting antibodies, as part of a transplant regimen (autologous bone marrow transplant or allogeneic bone marrow transplant or peripheral blood stem cell transplant) to improve the survival of transplant recipients.

Therapeutically effective antibody "fragments" refers to any portion of or derivative of an antibody that is capable of delivering substantially the same therapeutic effect as the whole antibody when administered to a patient having intermediate- or high-grade non-Hodgkin's lymphoma (NHL), or when used as part of a transplant regimen.

As the understanding of lymphoma improves and new histopathologic variations are diagnosed, new classification systems for the different types of lymphoma have emerged. In general, for the purposes of the methods described herein, intermediate- and high-grade lymphomas are defined as those designated in the "Working Formulation" established in 1982. This system includes as intermediate-grade lymphomas follicular large cell (FL), diffuse small cleaved cell (DSC), diffuse mixed small and large cell (DM), and diffuse large cell, cleaved or noncleaved (DL). As high-grade lymphomas, the system recognizes immunoblastic large cell (IBL), lymphoblastic, convoluted or nonconvoluted (LL), and small noncleaved cell, Burkitt's or non-Burkitt's (SNC).

Several classification systems have emerged since the proposed Working Formulation. For instance, a recent classification system proposed by European and American pathologists is called the Revised European American Lymphoma (REAL) Classification. Although this classification system does not use the terms "intermediate-" and "high-grade" NHL, it will be understood by those of skill in the art which lymphomas are typically characterized as "intermediate-" and "high-grade." For instance, "mantle cell lymphoma" as defined in the REAL classification system may appear as both indolent and more aggressive forms, and depending on the severity may be classified as an intermediate- or high-grade lymphoma.

For instance, the U.S. National Cancer Institute (NCI) has in turn divided some of the REAL classes into more clinically useful "indolent" or "aggressive" lymphoma designations. "Aggressive" lymphomas include diffuse mixed and large cell lymphoma, Burkitt's lymphoma/ diffuse small non-cleaved cell lymphoma, Lymphoblastic lymphoma, Mantle cell lymphoma and AIDS-related lymphoma. These lymphomas would therefor be considered at least "intermediate" or "high-grade," and would therefor benefit from the therapeutic methods of the present invention.

While strict classifications of some lymphomas may be difficult, the lymphomas treatable by the present invention are generally characterized by a high number of circulating B cells, possible bone marrow involvement, bulky disease, or the involvement of extralymphatic organ or sites.

Often, the patients which will most benefit from the disclosed therapeutic methods are those patients who are refractory to other types of treatments, or who have relapsed following other types of treatments, such as chemotherapy or radiotherapy. However, the monoclonal antibody treatments disclosed in the present invention will be beneficial also to newly diagnosed patients, and may have a synergistic effect in decreasing the chance of relapse if administered in conjunction with other conventional therapies.

For instance, the methods of the present invention include methods comprising the administration of both monoclonal antibodies (or fragments thereof) to CD20 along with a chemotherapeutic regimen. Depending on the particular patient, said chemotherapy may be administered simultaneously or sequentially in either order. "Simultaneously" means either concurrently or during the same time period such that the circulating half-lives of the therapeutic agents overlaps.

Chemotherapeutic regimens which may be combined with the antibody treatments of the present invention include CHOP, ICE, Mitozantrone, Cytarabine, DVP, ATRA, Idarubicin, hoelzer chemotherapy regime, La La chemotherapy regime, ABVD, CEOP, 2-CdA, FLAG & IDA with or without subsequent G-CSF treatment), VAD, M & P, C-Weekly, ABCM, MOPP and DHAP. The most preferred chemotherapeutic regimen is CHOP.

The primary anti-CD20 antibodies of the present invention are preferably human antibodies, or chimeric or humanized antibodies which are engineered with human constant region domains, such that the antibodies are able to stimulate human effector functions. A preferred antibody to be used in the methods of the present invention is Rituximab® (IDEC Pharmaceuticals, Inc.).

Rituximab® is one of a new generation of monoclonal antibodies developed to overcome limitations encountered with murine antibodies, including short half-life, limited ability to stimulate human effector functions, and immunogenicity. Rituximab® is a genetically engineered monoclonal antibody with murine light-and heavy-chain variable regions and human gamma I heavy-chain and kappa light-chain constant regions. The chimeric antibody is composed of two heavy chains of 451 amino acids and two light chains of 213 amino acids and has an approximate molecular weight of 145 kD.

Rituximab® is more effective than its murine parent in fixing complement and mediating ADCC, and it mediates CDC in the presence of human complement. The antibody inhibits cell growth in the B-cell lines FL-18, Ramos, and Raji, sensitizes chemoresistant human lymphoma cell lines to diphtheria toxin, ricin, CDDP, doxorubicin, and etoposide, and induces apoptosis in the DHL-4 human B-cell lymphoma line in a dose-dependent manner. In humans, the half-life of the antibody is approximately 60 hours after the first infusion and increases with each dose to 174 hours after the fourth infusion. The immunogenicity of the antibody is low; of 355 patients in seven clinical studies, only three (<1%) had a detectable anti-chimeric antibody (HACA) response.

The methods of the present invention may comprise administration of a radiolabeled antibody which binds to a protein on the surface of cancerous B cells. Such radiolabeled antibodies are preferably administered following administration of the human, chimeric or humanized antibody, which will decrease the amount of cancerous B cells in the bone marrow and lessen the likelihood of unwanted myeloablative suppression due to antibody binding to tumor cells in the marrow. Moreover, while CD20 is an ideal target for the immunotherapy of the present invention, it is possible that radiolabeled antibodies directed to other B cell surface antigens may also be used in the methods of the present invention. In particularly preferred embodiments, the radiolabeled antibodies are used in conjunction with unlabeled antibodies.

Approximately 80% of non-Hodgkin's lymphomas are B-cell malignancies and > 95% of these express the CD20 antigen on the cell surface. This antigen is an attractive target for immunotherapy because it is found exclusively on B cells, and not on hematopoietic stem cells, pro-B cells, normal plasma cells, or other normal tissues. It is not shed from the cell surface and does not modulate upon antibody binding.

The radiolabeled antibodies of the present invention may be labeled with any alpha or beta emitting radioisotope. However, a preferred isotope is ⁹⁰Y, and a preferred antibody is Y2B8. Y2B8 was engineered from the same murine antibody, 2B8, as was Rituximab®. The 2B8 antibody has also been conjugated to different radiolabels for diagnostic and therapeutic purposes. To this end, copending applications 08/475,813, 08/475,815 and 08/478,967, herein incorporated by reference in their entirety, disclose radiolabeled anti-CD20 conjugates for diagnostic "imaging" of B cell lymphoma tumors before administration of therapeutic antibody. For instance, the "In2B8" conjugate comprises the murine monoclonal antibody, 2B8, attached to Indium[111] (¹¹¹In) via a bifunctional chelator, i.e., MX-DTPA (diethylenetriaminepentaacetic acid), which comprises a 1:1 mixture of 1-isothiocyanatobenzyl-3-methyl-DTPA and 1-methyl-3-isothiocyanatobenzyl-DTPA. Indium-[111] is selected as a diagnostic radionuclide because it emits gamma radiation and finds prior usage as an imaging agent.

Patents relating to chelators and chelator conjugates are known in the art. For instance, U.S. Patent No. 4,831,175 of Gansow is directed to polysubstituted diethylenetriaminepentaacetic acid chelates and protein conjugates containing the same, and methods for their preparation. U.S. Patent Nos. 5,099,069, 5,246,692, 5,286,850, and 5,124,471 of Gansow also relate to polysubstituted DTPA chelates. These patents are incorporated herein in their entirety.

The specific bifunctional chelator used to facilitate chelation in applications 08/475,813, 08/475,815 and 08/478,967 was selected as it possesses high affinity for trivalent metals, and provides for increased tumor-to-non-tumor ratios, decreased bone uptake, and greater *in vivo* retention of radionuclide at target sites, i.e., B-cell lymphoma tumor sites. However, other bifunctional chelators are known in the art and may also be beneficial in tumor therapy.

Also disclosed in applications 08/475,813, 08/475,815 and 08/478,967 are radiolabeled therapeutic antibodies for the targeting and destruction of B cell lymphomas and tumor cells. In particular, the Y2B8 conjugate comprises the same anti-human CD20 murine monoclonal antibody, 2B8, attached to yttrium-[90] (⁹⁰Y) via the same bifunctional chelator. This radionuclide was selected for therapy for several reasons. The 64 hour half-life of ⁹⁰Y is long enough to allow antibody accumulation by the tumor and, unlike e.g. ¹³¹I, it is a pure beta emitter of high energy with no accompanying gamma irradiation in its decay, with a range of 100 to 1000 cell diameters. The minimal amount of penetrating radiation allows for outpatient administration of ⁹⁰Y-labeled antibodies. Furthermore, internalization of labeled antibodies is not required for cell killing, and the local emission of ionizing radiation should be lethal for adjacent tumor cells lacking the target antigen.

Because the ⁹⁰Y radionuclide was attached to the 2B8 antibody using the same bifunctional chelator molecule MX-DTPA, the Y2B8 conjugate possesses the same advantages discussed above, e.g., increased retention of radionuclide at a target site (tumor). However, unlike ¹¹¹In, it cannot be used for imaging purposes due to the lack of gamma radiation associated therewith. Thus, a diagnostic "imaging" radionuclide, such as ¹¹¹In, can be used for determining the location and relative size of a tumor prior to and/or following administration of therapeutic chimeric or ⁹⁰Y-labeled antibodies in the combined regimens of the invention. Additionally, indium-labeled antibody enables dosimetric assessment to be made.

Depending on the intended use of the antibody, i.e., as a diagnostic or therapeutic reagent, other radiolabels are known in the art and have been used for similar purposes. For instance, radionuclides which have been used in clinical diagnosis include ¹³¹I, ¹²⁵I, ¹²³I, ⁹⁹Tc, ⁶⁷Ga, as well as ¹¹¹In. Antibodies have also been labeled with a variety of radionuclides for potential use in targeted immunotherapy (Peirersz et al. (1987) The use of monoclonal antibody conjugates for the diagnosis and treatment of cancer. Immunol. Cell Biol. 65: 111-125). These radionuclides include ¹⁸⁸Re and ¹⁸⁶Re as well as ⁹⁰Y, and to a lesser extent ¹⁹⁹Au and ⁶⁷Cu. U.S. Patent No. 5,460,785 provides a listing of such radioisotopes and is herein incorporated by reference.

As reported in copending applications 08/475,813, 08/475,815 and 08/478,967 administration of the radiolabeled Y2B8 conjugate, as well as unlabeled chimeric anti-CD20 antibody (Rituximab®), resulted in significant tumor reduction in mice harboring a B cell lymphoblastic tumor. Moreover, human clinical trials reported therein showed significant B cell depletion in low-grade NHL lymphoma patients infused with Rituximab®. In fact, Rituximab® has recently been heralded the nation's first FDA-approved anti-cancer monoclonal antibody.

In addition, U.S. Application Serial No. 08/475,813, herein incorporated by reference, discloses sequential administration of Rituxan®, a chimeric anti-CD20, with both or either indium-labeled or yttrium-labeled murine monoclonal antibody for the treatment of low-grade NHL. Although the radiolabeled antibodies used in these combined therapies are murine antibodies, initial treatment with chimeric anti-CD20 sufficiently depletes the B cell population such that the HAMA response is decreased, thereby facilitating a combined therapeutic and diagnostic regimen. Moreover, it was shown in U.S. Application 08/475,813 that a therapeutically effective dosage of the yttrium-labeled anti-CD20 antibody following administration of Rituximab® is sufficient to (a) clear any remaining peripheral blood B cells not cleared by the chimeric anti-CD20 antibody; (b) begin B cell depletion from lymph nodes; or (c) begin B cell depletion from other tissues.

Autologous bone marrow transplantation is often a successful accompaniment to myeloablative therapy in helping to restore the immune system to patients who have undergone radiotherapy or chemotherapy. However, as discussed above, the patients who will benefit by the methods disclosed herein will often have lymphoma accompanied by bone marrow involvement. For such patients, there are often too many cancerous cells in the marrow to perform autologous transplantation.

When there is bone marrow involvement accompanying the intermediate- or high-grade lymphoma, such patients may benefit by prior treatment with human, chimeric or humanized anti-CD20 antibody before bone marrow harvesting in order to decrease the quantity of tumor cells in the bone marrow or stem cell preparation. In fact, Rituximab® can be administered at induction, in vivo purging, mobilization, conditioning, post-transplant reinfusion and at any other time during bone marrow or stem cell transplant for the purpose of improving the survival rate of transplant recipients. "Induction" is meant to refer to the initial therapies aimed at achieving induction of remission. Typically, induction involves the administration of some type of chemotherapy, i.e., CHOP.

The phrase "in vivo purging" is meant to refer to treatment particularly geared toward purging tumor cells from the bone marrow within the patient, although certainly such treatment might be beneficial for tumor cells in the peripheral blood and at other sites as well. Such a step may precede the harvest of bone marrow as a means of decreasing the number of tumor cells therein. Rituximab® and other chimeric lymphoma cell-depleting antibodies provide an advantage in this regard over radiolabeled antibodies in that they may be used to purge the bone marrow of cancerous cells without damaging healthy progenitors.

"Mobilization" refers to the process by which stem cells are mobilized to leave the bone marrow and enter the circulatory system, and provides an alternative to bone marrow harvest per se as a source of stem cells for transplantation. Mobilization is typically achieved by administering a short burst of chemotherapy and/or growth factors. The growth factor G-CSF is commonly used, but others may be used according to the knowledge of the skilled artisan.

Typically, during mobilization, stem cells are separated from blood (which is then put back into the patient), and the stem cells are frozen until the patient is ready to be reinfused. Ex vivo purging with Rituximab®, or other antibodies known in the art to be useful for this purpose, may then be used to deplete tumor cells in the stem cell preparation.

"Conditioning refers to a process by which the patient is prepared to receive the autologous bone marrow reinfusion or allogeneic transplant. This is typically accomplished with a very high dose of chemotherapy in order to deplete all cells, both healthy cells and tumor cells, from the bone marrow. Chemotherapeutic drugs that may be given at sufficiently high doses without risking the patient's life, e.g. cyclophosphamide, are known in the art.

Thus, with Rituximab® treatment at the various stages of transplantation, marrow may be harvested prior to myeloablative radiotherapy, and reintroduced subsequent to such therapy with less concern about reintroducing tumor cells originally harvested with the marrow back into the patient. Of course, the patient may then benefit by additional or subsequent treatment with chimeric anti-CD20 antibody as part of a maintenance regimen, or by administration of a radiolabeled antibody such as Y2B8 to further decrease the chance of relapse.

The methods of the present invention also encompass combined therapy comprising administration of at least one cytokine along with an anti-CD20 antibody or fragment thereof. Such a cytokine may be administered simultaneously or sequentially in any order. In particular, cytokines may be useful in upregulating the expression of CD20 on the surface of cancerous B cells prior to administration of the anti-CD20 antibody. Cytokines useful for this purpose include IL-4, GM-CSF and TNF-alpha, and possibly others.

Cytokines may also be administered simultaneously or within the same time frame in order to increase or control certain effector functions mediated by the therapeutic antibody. Cytokines useful for this purpose include interferon alpha, G-CSF and GM-CSF, and possibly others.

Preferred dosage regimens and exemplary embodiments will now be illustrated by way of the following data.

### Single-Agent Studies

In a study conducted in Europe and Australia, alternative dosing schedules were evaluated in 54 relapsed or refractory intermediate- or high-grade NHL patients (Coiffier B, Haioun C, Ketterer N, Engert A, Tilly H, Ma D, Johnson P, Lister A, Feuring-Buske M, Radford JA, Capdeville R, Diehl V, Reyes F. Rituximab (anti-CD20 monoclonal antibody) for the treatment of patients with relapsing or refractory aggressive lymphoma: a multicenter phase H study. Blood 1998; 92:1927-1932).

Rituximab® was infused at 375 mg/m² weekly for 8 doses or at 375 mg/m2 once followed by 500 mg/m² weekly for 7 doses. The ORR was 31 %; (CR 9%, PR 22%) no significant difference between the dosing regimens was observed. Patients with diffuse large-cell lymphoma (N = 30) had an ORR of 37% and those with mantle-cell lymphoma (N = 12) had an ORR of 33%.

### Treatment of Bulky Disease

Contrary to early assumptions about antibody therapy being useful only in micrometastatic disease, Rituximab® is quite active in high bulk disease. In a separate study, 31 patients with relapsed or refractory, bulky low-grade NHL (single lesion of > 10 cm in diameter) received 375 mg/m² Rituximab as four weekly infusions. Twelve of 28 evaluable patients (43%) demonstrated a CR (1, 4%) or PR (11, 39%) (Davis T, White C, Grillo-López A, Velasquez W, Link B, Maloney D, Dillman R, Williams M, Mohrbacher A, Weaver R, Dowden S, Levy R. Rituximab: First report of a Phase II (PII) trial in NHL patients (pts) with bulky disease. Blood 1998; 92 (10 Suppl 1):414a).

This suggests that with the appropriate dosages depending on the extent of disease and the number of circulating tumor cells (i.e., such as the increased dosages described above), Rituximab® therapy will also be useful for more aggressive intermediate- or high-grade NHLs accompanied by bulky disease.

### Combination of Rituximab® and CHOP Chemotherapy

In another study, 31 patients with intermediate- or high-grade NHL (19 females, 12 males, median age 49) received Rituximab® on Day 1 of each of six 21 - day cycles of CHOP Link B, Grossbard M, Fisher R, Czuczman M, Gilman P, Lowe A, Vose J. Phase II pilot study of the safety and efficacy of rituximab in combination with CHOP chemotherapy in patients with previously untreated- or high-grade NHL. Proceedings of the American Society of Clinical Oncology 1998; 17:3a). Of 30 evaluable patients, there were 19 CR (63%) and 10 PR (33%), for an ORR of 96%. This regimen was considered well tolerated and may result in higher response rates than with Rituximab or CHOP alone.

The NCI Division of Cancer Treatment and Diagnosis is collaborating with IDEC Pharmaceuticals Corporation to explore Rituximab® treatment in other indications. A Phase II trial of CHOP versus CHOP and Rituximab® is being conducted by ECOG, CALGB, and SWOG in older patients (> 60 years) with mixed, diffuse large cell, and immunoblastic large cell histology NHL (N = 630 planned). This study includes a secondary randomization to maintenance with Rituximab® versus nonmaintenance.

A Phase III trial of Rituximab® and CHOP in 40 patients with previously untreated mantle-cell lymphoma is also ongoing at the Dana Farber Institute. Rituximab® is administered on Day 1 and CHOP is given on Days 1 - 3 every 21 days for 6 cycles. Accrual for this study has been completed. A Phase II trial of CHOP followed by Rituximab in newly diagnosed follicular lymphoma conducted by SWOG has also been completed. Results of these two trials are being analyzed. A Phase II trial of CHOP and Rituximab versus CHOP alone in HIV-related NHL conducted by the AIDS Malignancy Consortium is ongoing; 120 patients are planned.

### Rituximab® after Myeloablative Therapy Relapse

Rituximab® has shown promising early results in patients with relapsed intermediate-grade NHL after high-dose therapy with autologous PBSC support. Six of seven patients responded (1 CR and 5 PR) and one patient had stable disease; therapy was well tolerated (Tsai, D, Moore H, Porter D, Vaughn D, Luger S, Loh R, Schuster S, Stadtmauer E. Progressive intermediate grade non-Hodgkin's lymphoma after high dose therapy and autologous peripheral stem cell transplantation (PSCT) has a high response rate to Rituximab. Blood 1998; 92:415a, #1713).

### Statements of Invention

1. A method for treating or alleviating the symptoms of intermediate- or high-grade non-Hodgkins lymphoma comprising administering to a patient a therapeutically effective amount of an anti-CD20 antibody or a therapeutically effective fragment thereof.
2. The method of 1, wherein said non-Hodgkins lymphoma is selected from the group of classifications consisting of follicular large cell (FL), diffuse small cleaved cell (DSC), diffuse mixed small and large cell (DM), diffuse large cleaved cell (DL-C), diffuse noncleaved large cell (DL), immunoblastic large cell (IBL), lymphoblastic convoluted (LL-C), lymphoblastic nonconvoluted (LL), small noncleaved cell - Burkitt's (SNC-B), small noncleaved cell - non-Burkitt's (SNC), mantle cell lymphoma and AIDS related lymphoma.
3. The method of 2, wherein said non-Hodgkin's lymphoma is accompanied by bulky disease.
4. The method of 1, wherein said patient is refractory to other treatments.
5. The method of 4, wherein said patient is refractory to chemotherapy or radiotherapy.
6. The method of 1, wherein said patient has relapsed after previous treatment for non-Hodgkins's lymphoma.
7. The method of 6, wherein said patient has relapsed following chemotherapy or radiotherapy.
8. The method of 1, further comprising administering to said patient a chemotherapeutic regimen.
9. The method of 8, wherein said chemotherapy is administered simultaneously or sequentially in either order.
10. The method of 9, wherein said chemotherapeutic regimen is selected from the group consisting of CHOP, ICE, Mitozantrone, Cytarabine, DVP, ATRA, Idarubicin, hoelzer chemotherapy regime, La La chemotherapy regime, ABVD, CEOP, 2-CdA, FLAG & IDA with or without subsequent G-CSF treatment), VAD, M & P, C-Weekly, ABCM, MOPP and DHAP.
11. The method of 10, wherein said chemotherapeutic regimen is CHOP.
12. The method of 1, wherein said antibody is a chimeric antibody.
13. The method of 12, wherein said chimeric antibody is Rituximab®.
14. The method of 1 further comprising administration of a radiolabeled antibody which binds to a protein on the surface of cancerous B cells.
15. The method of 14, wherein said radiolabeled antibody is an anti-CD20 antibody.
16. The method of 15, wherein said antibody is Y2B8.
17. The method of 1, wherein said intermediate- or high-grade lymphoma is accompanied by bone marrow involvement.
18. The method of 17, wherein said method further comprises bone marrow or stem cell transplantation.
19. The method of 18, wherein said bone marrow or stem cell transplantation is autologous.
20. The method of 19, wherein said patient is treated with chimeric anti-CD20 antibody prior to and/or subsequently to the harvesting or transplantation of bone marrow or stem cells.
21. The method of 17, wherein said antibody is a chimeric anti-CD20 antibody.
22. The method of 21, further comprising administration of a radiolabeled antibody subsequently to said chimeric anti-CD20 antibody.
23. The method of 22, wherein said antibody is an anti-CD20 antibody.
24. The method of 23, wherein said antibody is Y2B8.
25. The method of 1, further comprising administration of at least one cytokine.
26. The method of 1, wherein said cytokine is administered prior to said anti-CD20 antibody in order to upregulate the expression of CD20 on the surface of cancerous B cells.
27. The method of 25 wherein said cytokine is selected from the group consisting of interferon alpha, G-CSF and GM-CSF.
28. The method of 26, wherein said cytokine is selected from the group consisting of IL-4, GM-CSF and TNF-alpha.
29. The method of 19, wherein said anti-CD20 antibody is administered to said patient at the induction satge of a bone marrow or stem cell transplant regimen.
30. The method of 19, wherein said anti-CD20 antibody is administered to said patient at the in vivo purging stage of a bone marrow or stem cell transplant regimen.
31. The method of 19, wherein said anti-CD20 antibody is administered to said patient at the mobilization stage of a bone marrow or stem cell transplant regimen.
32. The method of 19, wherein said anti-CD20 antibody is administered to said patient at the conditioning stage of a bone marrow or stem cell transplant regimen.
33. The method of 19, wherein said anti-CD20 antibody is administered to said patient at the post-transplant reinfusion stage of a bone marrow or stem cell transplant regimen.
34. The method of 19, wherein said anti-CD20 antibody is administered to said patient as part of a post-transplant maintenance regimen.

## Claims

1. An anti-CD20 antibody or a therapeutically effective fragment thereof, for use in a method for treating or alleviating the symptoms of intermediate-or high-grade non-Hodgkins lymphoma, the method comprising administering to a patient a therapeutically effective amount of the antibody or fragment thereof.

2. The antibody or fragment thereof for use according to claim 1, wherein said non-Hodgkins lymphoma is selected from the group of classifications consisting of diffuse large cell lymphoma, follicular large cell (FL), diffuse small cleaved cell (DSC), diffuse mixed small and large cell (DM), , immunoblastic large cell (IBL), lymphoblastic convoluted (LL-C), lymphoblastic nonconvoluted (LL), small noncleaved cell-Burkitt's (SNC-B), small noncleaved cell-non-Burkitt's (SNC), mantle cell lymphoma and AIDS related lymphoma

3. The antibody or fragment for use according to claim 2, wherein said non-Hodgkin's lymphoma is accompanied by bulky disease.

4. The antibody or fragment for use according to any one or claims 1 to 3, wherein the patient is of > 60 years.

5. The antibody or fragment for use according to claim 1, wherein said patient is refractory to other treatments, for example chemotherapy or radiotherapy.

6. The antibody or fragment for use according to claim 1, wherein said patient has relapsed after previous treatment for non-Hodgkins lymphoma, for example following chemotherapy or radiotherapy.

7. The antibody or fragment for use according to any one of the preceding claims, the method further comprising administering to said patient a chemotherapeutic regimen, optionally wherein said chemotherapy is administered simultaneously or sequentially in either order.

8. The antibody or fragment for use according to claim 7, wherein said chemotherapeutic regimen is selected from the group consisting of DHAP, VAD, CHOP, ICE, Mitozantrone, Cytarabine, DVP, ATRA, Idarubicin, hoelzer chemotherapy regime, La La chemotherapy regime, ABVD, CEOP, 2-CdA, FLAG & IDA with or without subsequent G-CSF treatment), M & P, C-Weekly, ABCM and MOPP.

9. The antibody or fragment for use according to any one of the preceding claims, wherein said antibody is a chimeric antibody.

10. The antibody or fragment for use according to claim 9, wherein said chimeric antibody is rituximab.

11. The antibody or fragment for use according to any one of claims 1 to 10, wherein said intermediate-or high-grade lymphoma is accompanied by bone marrow involvement, and, wherein said method optionally further comprises bone marrow or stem cell transplantation, wherein said bone marrow or stem cell transplantation may be autologous, wherein if the bone marrow or stem cell transplantation is autologous said patient is optionally treated with chimeric anti-CD20 antibody prior to and/or subsequently to the harvesting or transplantation of bone marrow or stem cells.

12. The antibody or fragment for use according to claim 11, wherein in the method said anti-CD20 antibody is administered to said patient at the induction stage of a bone marrow or stem cell transplant regimen, said anti-CD20 antibody is administered to said patient at the *in vivo* purging stage of a bone marrow or stem cell transplant regimen, said anti-CD20 antibody is administered to said patient at the mobilization stage of a bone marrow or stem cell transplant regimen, said anti-CD20 antibody is administered to said patient at the conditioning stage of a bone marrow or stem cell transplant regimen, said anti-CD20 antibody is administered to said patient at the post-transplant reinfusion stage of a bone marrow or stem cell transplant regimen, or said anti-CD20 antibody is administered to said patient as part of a post-transplant maintenance regimen.

13. The antibody or fragment for use according to claim 11, wherein in the method the patient is treated with chimeric anti-CD20 antibody and the method further comprises administration of a radiolabeled antibody subsequently to said chimeric anti-CD20 antibody, wherein the radiolabeled antibody is optionally an anti-CD20 antibody, and may be Y2B8.

14. The antibody or fragment for use according to claim 1 further comprising administration of a radiolabeled antibody which binds to a protein on the surface of cancerous B cells, wherein said radiolabeled antibody is optionally an anti-CD20 antibody, and may be Y2B8.

15. The antibody or fragment for use according to claim 1, the method further comprising administration of at least one cytokine, optionally wherein said cytokine is administered prior to said anti-CD20 antibody in order to upregulate the expression of CD20 on the surface of cancerous B cells, optinally wherein the cytokine is selected from the group consisting of interferon alpha, G-CSF and GM-CSF or optionally wherein the cytokine is selected from the group consisting of IL-4, GM-CSF and TNF-alpha.
